# EUROPEAN PATENT SPECIFICATION

(11) **EP 0 814 819 B1**
(45) Date of publication and mention of the grant of the patent: **14.07.2004**
(21) Application number: 96908759.2
(22) Date of filing: 12.03.1996
(51) Int. Cl.: A61K 35/30, A61K 35/48, A61P 25/00

(54) **SERTOLI CELLS AS TRANSPLANTATION FACILITATOR FOR CELL TRANSPLANTATION**
SERTOLIZELLEN ALS TRANSPLANTATIONUNTERSTÜTZER BEI ZELLTRANSPLANTATIONEN
CELLULES DE SERTOLI EMPLOYEES COMME FACILITATEUR DE TRANSPLANTATION CELLULAIRE

(30) Priority: 13.03.1995 US 402387
(43) Date of publication of application: 07.01.1998
(73) Proprietor: UNIVERSITY OF SOUTH FLORIDA, Tampa, FL 33620-7900 (US)
(72) Inventor: SANBERG, Paul, R., Spring Hill, FL 34610 (US); CAMERON, Don, F., Lutz, FL 33549 (US); BORLONGAN, Cesario, V., Lutz, FL 33549 (US); HELLER, Richard, Brandon, FL 33511 (US)
(74) Representative: Davies, Gregory Mark
(86) International application number: PCT/US1996/003337
(87) International publication number: WO 1996/028174

(56) References cited:
- WO-A-92/06702
- SELAWRY H P ET AL: "Production of a factor, or factors, suppressing IL-2 production and T cel proliferation by Sertoli cell-enriched preparations. A potentia role for islet transplantation in an immunologically privileged site." TRANSPLANTATION, (1991 NOV) 52 (5) 846-50., XP000602288
- WYATT CR ET AL: "Suppression of lymphocyte proliferation by proteins secreted by cultured Sertoli cells" J REPRODUC IMMUNOL, vol. 14, 1988, pages 27-40, XP000983203
- DE CESARIS P ET AL.: "Immunosuppressive molecules produced by Sertoli cells cultured in vitro: biological effects on lymphocytes" BIOCHEM BIOPHYS RES COMMUN, vol. 186, no. 3, 14 August 1992 (1992-08-14), pages 1639-1646, XP000942038
- SANBERG P R ET AL: "SERTOLI CELLS - AN ALTERNATIVE CELL SOURCE FOR NEURAL TRANSPLANTATION IN PARKINSONS-DISEASE" EXPERIMENTAL NEUROLOGY, ( OCT 1995 ) VOL. 135, NO. 2, PP. 169. ISSN: 0014-4886., XP000983205
- SANBERG, P. R. (1) ET AL: "Functional recovery in hemiparkinsonian rats following neural transplantation of testis-derived Sertoli cells." SOCIETY FOR NEUROSCIENCE ABSTRACTS, (1995) VOL. 21, NO. 1-3, PP. 317. MEETING INFO.: 25TH ANNUAL MEETING OF THE SOCIETY FOR NEUROSCIENCE SAN DIEGO, CALIFORNIA, USA NOVEMBER 11-16, 1995, XP000983272
- ROBERTS K P ET AL: "IMMORTALIZATION AND CHARACTERIZATION OF A SERTOLI CELL LINE FROM THE ADULT RAT" BIOLOGY OF REPRODUCTION,US,SOCIETY FOR THE STUDY OF REPRODUCTION, CHAMPAIGN, IL, vol. 53, no. 6, 1 December 1995 (1995-12-01), pages 1446-1453, XP000602206 ISSN: 0006-3363
- SANBERG PAUL R ET AL: "Testis-derived Sertoli cells survive and provide localized immunoprotection for xenografts in rat brain." NATURE BIOTECHNOLOGY, vol. 14, no. 13, 1996, pages 1692-1695, XP000942044 ISSN: 1087-0156
- BORLONGAN CV ET AL: "CNS immunological modulation of neural graft rejection and survival" NEUROLOGICAL RESEARCH, vol. 18, August 1996 (1996-08), pages 297-304, XP000983230
- CELL TRANSPLANTATION, Volume 2, issued 1993, SELAWRY et al., "Sertoli Cell-Enriched Fractions in Successful Islet Cell Transplantation", pages 123-129, XP000602285
- THE JOURNAL OF NEUROSCIENCE, Volume 13, Number 6, issued June 1993, SAGEN et al., "Transplants of Immunologically Isolated Xenogeneic Chromaffin Cells Provide a Long-Term Source of Pain-Reducing Neuroactive Substances", pages 2415-2423, XP002940605
- SOCIETY FOR NEUROSCIENCE ABSTRACTS, Volume 21, Part 1, issued November 1995, SANBERG et al., "Functional Recovery in Hemiparkinsonian Rats Following Neural Transplantation of Testis-Derived Sertoli Cells", page 317, Abstract 133.12, XP000983272
- EOS-RIVISTA DI IMMUNOLOGIA ED IMMUNOFARMACOLOGIA, Volume 12, Number 2, issued 1992, DeCESARIS et al., "Inhibition of Lymphocyte Activation by Sertoli Cell Immunosuppressive Factor(s)", page 86, XP002940607
- NATURE, Volume 377, issued 19 October 1995, BELLGRAU et al., "A Role for CD95 Ligand in Preventing Graft Rejection", pages 630-632, XP002940606
- CRITICAL REVIEWS IN NEUROBIOLOGY, Volume 8, Number 3, issued 1994, KOUTOUZIS et al., "Cell Transplantation for Central Nervous System Disorders", pages 125-162, XP002940608

## Description

### TECENICAL FIELD

The present invention relates to methods of transplanting cells to create a localized immunosuppressive and trophic effect in tissue.

### BACKGROUND OF THE INVENTION

Transplantation of cells and tissues is being utilized therapeutically in a wide range of disorders including but not limited to from cystic fibrosis (lungs), kidney failure, degenerative heart diseases to neurodegenerative disorders.

As an example, the central nervous system (CNS) (brain and spinal cord) has poor regenerative capacity which is exemplified in a number of neurodegenerative disorders. An example of such a disorder is Parkinson's disease. The preferred pharmacotherapy for Parkinson's disease is L-dopa which helps the symptoms of this disease in humans. However, the neuropathological damage and the debilitating progression is not reversed by this treatment protocol.

Laboratory and clinical studies have shown the transplantation of cells into the CNS is a potentially significant alternative therapeutic modality for neurodegenerative disorders such as Parkinson's disease (Wictorin et al., 1990; Lindvall et al., 1990; Sanberg et al., 1994; Bjorklund and Stenevi, 1985; Freeman et al., 1994). In some cases, transplanted neural tissue can survive and form connections with the CNS of the recipient (i.e. the host) (Wictorin et al., 1990). When successfully accepted by the host, the transplanted tissue (i.e. the graft) has been shown to ameliorate the behavioral deficits associated with the disorder (Sanberg et al., 1994). The obligatory step for the success of this kind of treatment is the prevention of graft rejection

### (i.e. graft acceptance).

Currently, fetal neural tissue is the primary graft source for neural transplantation (Lindvall et al., 1990; Bjorklund, 1992; Isacson et al., 1986; Sanberg et al., 1994). Other viable graft sources include adrenal chromaffin cells and various cell types that secrete neural growth factors and trophic factors. The field of neural tissue transplantation as a productive treatment protocol for neurodegenerative disorders has received much attention resulting in its progression to clinical trials. Preliminary results and clinical observations are promising although the graft rejection phenomenon remains a problem.

Recently, studies have suggested that Sertoli cells, when simultaneously transplanted with pancreatic islet cell into the diabetic rat, act as an effective local immunosuppressant on the host tissue (Selawry and Cameron, 1993). As a result, the graft is not rejected and the islets remain viable allowing the transplanted β-cells to function normally and produce insulin for an indefinite period of time. As a result, the accepted graft overcomes the primary physiological dysfunction of hyperglycemia thereby alleviating the related complications of this endocrine disorder. This cell transplantation protocol is accomplished without prolonged systemic immunosuppression, otherwise necessary when islets are transplanted without Sertoli cells.

In general, systemic immunosuppression is necessary if successful transplantation is to be achieved in humans. Immunosuppression of the entire body (i.e. systemic) can result, eventually, in graft acceptance. It is acquired, however, by placing the individual at medical risk making the immunosuppressant therapy itself more of a liability than a benefit in some cases. For a lack of a better immunosuppressant treatment, systemic immunosuppressants, with Cyclosporine-A (CsA) as the treatment choice, have been used as adjunctive therapy in neural and other transplantation protocols (Sanberg et al., 1994; Freeman et al., 1994; Borlongan et al., 1995). Arguably, systemic CsA treatment may be contraproductive to successful graft acceptance in the CNS because of its systemic effect and because CsA itself has been shown to cause detrimental side effects and may, in fact, be cytotoxic to neural tissues (Berden et al., 1985; de Groen et al., 1984).

It would be desirable to enhance the productive cell transplantation techniques already utilized for neurodegenerative disorders, such as Parkinson's disease, in ways which would more effectively slow the neurodegenerative disease process, more actively promote the re-establishment of normal neural tissue physiology and better alleviate the functional disabilities associated with the neural tissue dysfunction. Likewise, it would be desirable to avoid systemic immunosuppression with the ability to locally immunosuppress (i.e. at the graft site) by an immunosuppressant which is biologically tolerated by the host. Sertoli cells may provide this desired option since it is clear from the diabetic studies, as summarised above, that co-transplantation with Sertoli cells will deliver local immunosuppression and promote, therefore, efficient graft acceptance and functional restoration ofthe tissue-related dysfunction.

### SUMMARY OF THE INVENTION AND ADVANTAGES

In accordance with the present invention, there is provided a composition containing Sertoli cells and neural or adrenal chromaffin cells.

Further provided by the present invention is use of a composition containing Sertoli cells and neural or adrenal chromaffin cells for the manufacture of a medicament for use in producing a sustained localised immunosuppressive effect and trophic effect in the central nervous system of a subject, such as a human.

A further embodiment of the present invention provides use of a composition containing Sertoli cells and neural or adrenal chromaffin cells for the manufacture of a medicament for treating a neurological disorder.

### BRIEF DESCRIPTION OF THE DRAWINGS

Other advantages of the present invention will be readily appreciated as the same becomes better understood by reference to the following detailed description when considered in connection with the accompanying drawings wherein:

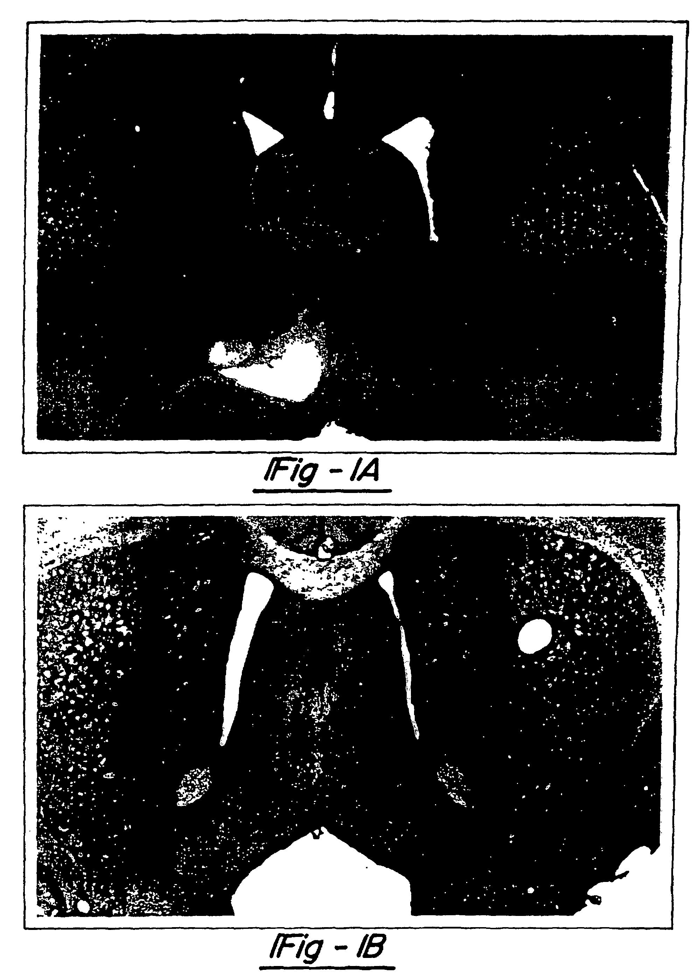

Figures 1A-B shows photomicrographs of two representative brain sections wherein the sections were stained with Lectin, the left side of each brain corresponds to the chromaffin cells transplanted side while the right side of each brain corresponds to the chromaffin cells with Sertoli cells the co-transplanted side;
Figures 2A-D show a higher magnification from the photomicrograph of Figure 1 of the transplant sites of brain sections illustrated in Figure 1; panels A and C show the chromaffin cells transplanted side while panels B and D show the side co-transplanted chromaffin cells with Sertoli cells;

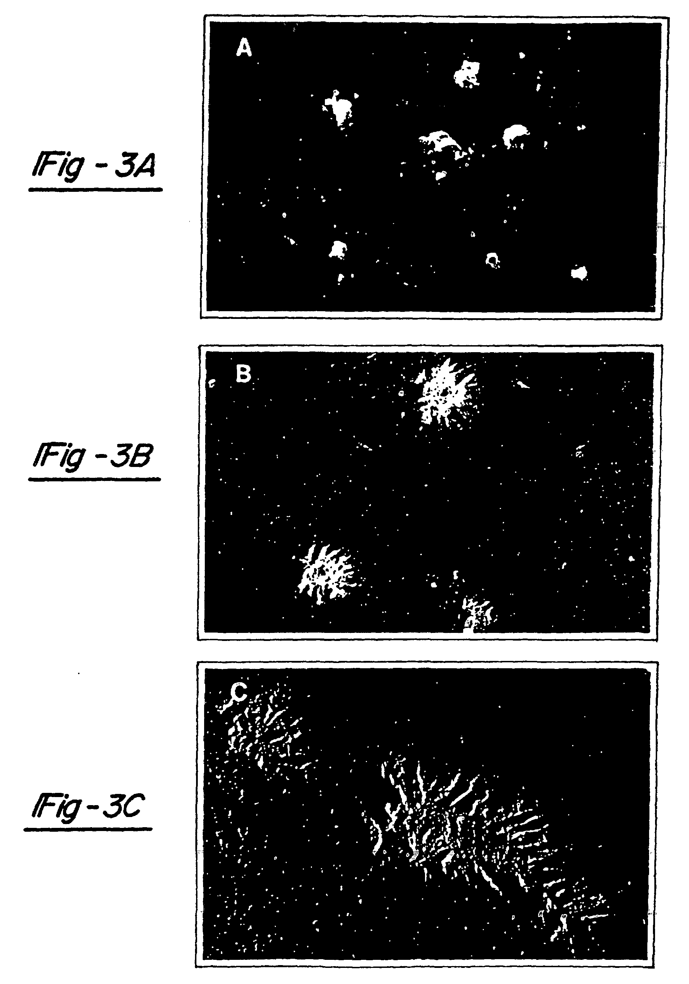

Figure 3A-C are light micrographs illustrating cells from the ventral mesencephalon of fetal rats (VM) isolated and cultured for seven days in control medium (CM) or Sertoli cell pre-conditioned medium (SCM) and photographed with darkfield, interference contrast optics, wherein (A) depicts VM cells incubated in CM showing no evidence of stimulation or differentiation, (B) depicts VM cells incubated in SCM appearing highly stimulated, and (C) at higher magnification, depicts VM cells incubated in SCM exhibiting neurite outgrowth;
Figure 4A-B are electron micrographs illustrating the cellular structure and tissue architecture of the Sertoli cell/chromaffin cell co-grafts in the striatum of the brain, wherein (**A**) depicts electron dense chromaffin cells (arrows) easily identified because of the inclusion of secretory granules unique to these cells, and (**B**) shows the boxed area in (**A**) at higher magnification, with higher resolution, Sertoli cells (arrows) are seen immediately adjacent to the electron dense chromaffin cells;

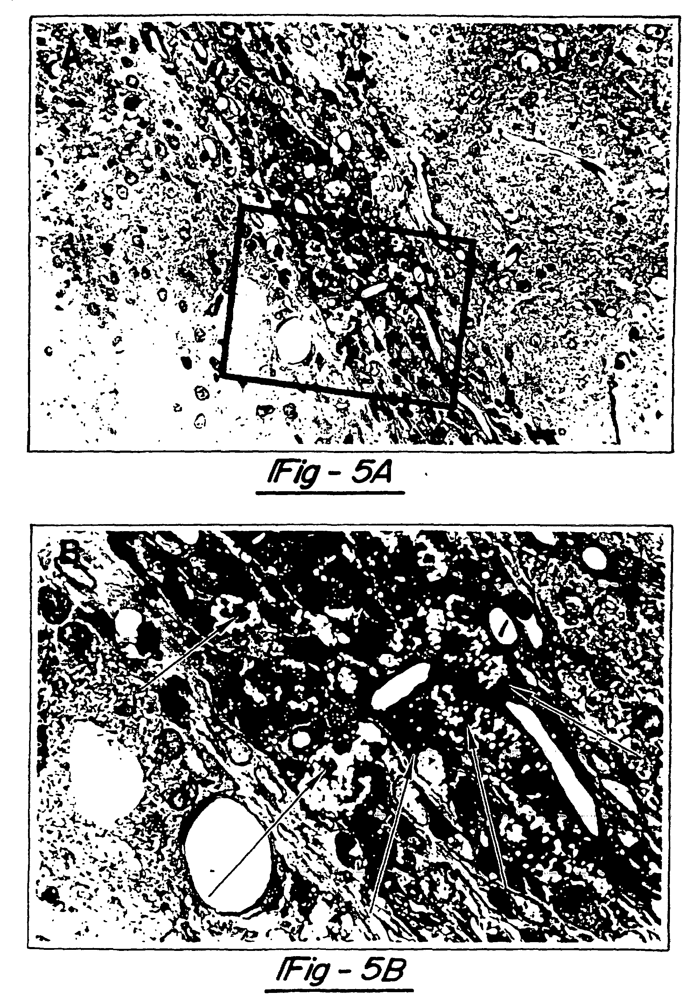

Figure 5A-B are electron micrographs illustrating (**A**) the striatum of the brain shows the penetration tract (arrows) and the site of Sertoli cell transplantation, and (**B**) shows the boxed area in (**A**) at higher magnification, with higher resolution, Sertoli cells (arrows) are easily identified because of the 1µ latex bead inclusions which were loaded into the cells prior to transplantation; and

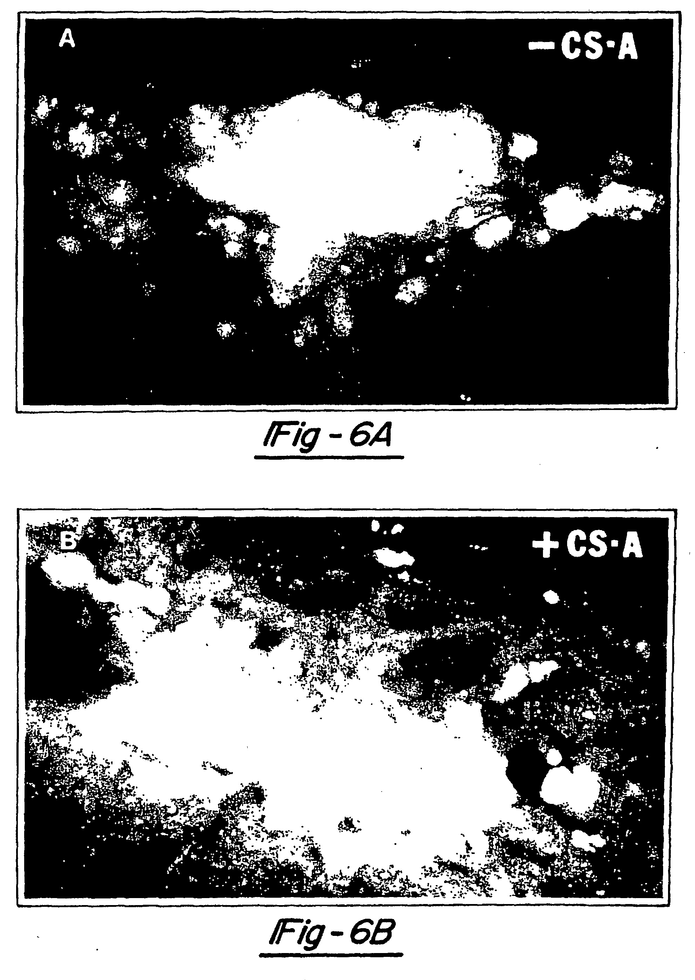

Figure 6A-B are two light micrographs illustrating grafted Sertoli cells *in situ* labeled with a florescent tag (DiI) prior to their transplantation into the striatum of the brain wherein (**A**) depicts viable, florescent Sertoli cells in a rat host that had not received immunosuppression therapy with Cyclosporine A (CsA), and (B) shows viable, florescent Sertoli cells in the rat host that had received cyclosporine A immunosuppression therapy.

### DETAILED DESCRIPTION OF THE INVENTION

The present invention provides a composition containing Sertoli cells and neural or adrenal chromaffin cells. The composition can be used to produce a sustained, localised immunosuppressive effect and trophic effect in tissue.

By sustained localised immunosuppressive effect, it is meant that the transplanted Sertoli cells will suppress the immunological response ordinarily mounted by the host tissue to the intrusion of foreign entities such as transplanted cells and that the immunosuppression will occur at the graft site (local) rather than by generalised immunosuppression of the entire body (systemic) which occurs with the ordinary methods of immunosuppression by agents such as CsA.

By tissue, it is meant any form of tissue including, but not limited to, cells, blood, organs, and disassociated cells.

In a preferred embodiment, the transplanted cells (which are intended to replace the dysfunctional cells or in some way alleviate tissue dysfunction) can avoid being rejected and thereby survive and functionally integrate into the host tissue. In the CNS, this will promote re-establishment of normal neural tissue function and thereby ameliorate the behavioral and functional deficits associated with the neurological and/or neurodegenerative disorder being treated.

With local immunosuppression by a Sertoli cell-derived immunosuppressant agent (which is now partially characterized), there would be no successful antibody or cellular immunological attack waged against the transplanted cells, including the Sertoli cells themselves. Additionally, since the immunosuppression is local and by a biologically tolerable agent, the side effects associated with both systemic immunosuppression and cytotoxicity of agents such as CsA would be avoided. Hence, the method of Sertoli cell transplantation provides a significant improvement over the use of systemic immunosuppression with CsA as the necessary adjunctive therapy to transplantation as shown in the example below.

The localized immunosuppression by a Sertoli cell-derived immunosuppressant agent can facilitate the survival of both xenografts and allografts. With allografts, co-transplantation with Sertoli cells should provide localized immunosuppression as to eliminate the need for systemic immunosuppression. With xenografts, co-transplantation with Sertoli cells may provide sufficient local immunosupression so as to eliminate the need for systemic immunosupression or the Sertoli cells may be used in combination with a systemic immunosuppressant to prevent rejection thereby reducing the dosage of systemic immunosuppressant required. When co-transplanted, the Sertoli cells not only provide immunosuppression, but provide regulatory, nutritional, and other factors designated as trophic support to the co-transplanted tissue. Therefore, the Sertoli cells will not only provide inhibition of the immune response, but will allow enhanced growth and viability of allografts and xenografts by concommitant trophic support.

The source of Sertoli cells is by primary cell isolation from the mammalian testis. The protocol for harvesting the cells is well-defined (Cameron and Muffly, 1991; Griswold, 1992) and considered a routine methodology. In most of the published reports of Sertoli cell co-transplantation, cells are derived from the rat (Selawry and Cameron, 1993). Although rat Sertoli cells are utilized in the following examples, it is contemplated that the method of the present invention can be used with Sertoli cells from any suitable mammalian source. A preferred source of Sertoli cells for use with mammals, such as humans, are porcine Sertoli cells. However, if available and suitable, human Sertoli cells may be utilized.

In one embodiment, the Sertoli cells are co-transplanted with the selected neural tissue into the CNS by intracranial infusion (Sanberg et al., 1995).

By proximate to the tissue, it is meant that the Sertoli cells are placed in general proximity to the selected tissue such as the neural tissue. Generally, this means that the Sertoli cells can be infused or transplanted into any mammal so as to become located in proximity to the selected tissue. For example, the location can be any site where there is neural tissue such as the CNS, PNS, or fluids which bathe neural tissue such as cerebral spinal fluid and blood, blood vessels.

The proximity of the Sertoli cells to the neural tissue is determined by the specific neural cells and function sought to be restored in a given transplantation.

The source of neural cells for transplantation depends on the neurological disorder being treated. For example, Parkinson's disease is treated with ventral mesencephalic tissue (Lindvall et al, 1990) or chromaffin cells (Lindvall et al, 1987), Huntington's disease is treated with striatal lateral eminence cells (Isacson et al, 1986) and neurological pain is treated with adrenal chromaffin cells (Sagen et al, 1993). Other tissue types experimentally transplanted into specific animal models of human neurodegenerative disorders are summarised elsewhere (Dunnett and Bjorklund, 1994) and provide detailed descriptions ofcell isolation and transplantation methods.

The present invention also teaches use of a composition containing Sertoli cells and neural or adrenal chromaffin cells for the manufacture of a medicament for use in producing a sustained localised immunosuppressive effect and trophic effect in the central nervous system (CNS) of a subject, such as a human. The cells secrete trophic factors *in* *situ,* for treating neurological disorders including epilepsy, stroke, Huntington's disease, head injury, spinal injury, pain, Parkinson's disease, myelin deficiencies, muscular dystrophy and other neuromuscular disorders, neurological pain, amyotrophic lateral sclerosis, Alzheimer's disease, and affective disorders of the brain.

The following examples demonstrate the methods of use of the present invention as well as efficacy for producing a sustained localized immunosuppressive effect.

### EXAMPLE 1

### Materials and Methods

Sprague-Dawley male rats, six weeks old, which were obtained from Harlan Sprague-Dawley, Inc. (Indianapolis, IN), were used. The animals were housed in individual Plexiglas cages in a room with controlled humidity and temperature and under a 12 hour light-dark cycle. Food and water were freely available *ad lib*.

### Cell Culture

Bovine chromaffin cells were obtained from the laboratory of Dr. Jaqueline Sagen at the University of Chicago at Illinois. Upon arrival, cells were plated using DMEM-F12 media with serum. Cell counts using the tryphan blue method revealed a total of 8X 10⁻⁶ per ml of surviving cells. A 95% viability of chromaffin cells were measured at the day of arrival and during the day of transplantation. Half of the chromaffin cells solution was co-cultured overnight with Sertoli cells.

The preparation of Sertoli cells is according to the method described by Selawry & Cameron (Selawry and Cameron, 1993).

Specifically, the testes were removed, chopped into several pieces, and placed in a 50 ml conical tube containing 50 ml of Ham's F12/DMEM media. The pieces were washed once by centrifugation at 800 X g for two minutes. The supernatant was aspirated, and the tissue resuspended in 40 ml of media containing 40 mg trypsin and 0.8 mg DNase in a sterile 250 ml Erlenmeyer flask. The flask was placed in an 37°C oscillating incubator at 60-90 osc/minutes for 30 minutes. This step removed Leydig cells.

The tubules were then transferred to a 50 ml conical tube, and centrifuged at 800 X g for two minutes. The supernatant fraction was aspirated, and the pellet resuspended in 40 ml of 1 M glycine, 2 mM EDTA containing 0.01% soybean trypsin inhibitor and 0.8 mg DNase, and incubated at room temperature for ten minutes. This step lysed any residual Leydig cells. The cells were washed by centrifugation for two minutes, and the step repeated twice, or until the media was no longer cloudy. The pellet was resuspended by gentle homogenization with a glass Pasteur pipet in 40 ml of media containing 20 mg collagenase in an Erlenmeyer flask, and incubated at 37°C for five minutes with 60-90 osc/minutes.

The cell suspension was centrifuged at 800 X g for two minutes, and the pellet was resuspended by gentle homogenization with a Pasteur pipet in 40 ml media containing 40 mg collagenase and 0.2 mg DNase, and incubated in an Erlenmeyer flask at 37°C for 30 minutes with 60-90 osc/minutes. The cells were then washed by centrifugation for two minutes, and the process repeated at least three times to eliminate peritubular cells. The cells were resuspended by gentle homogenization with a Pasteur pipet in 40 ml media containing 40 mg hyaluronidase and 0.2 mg of DNase, and incubated at 37°C for 30 minutes with 60-90 osc/min. The cells were pelleted by soft centrifugation for two minutes, and washed at least five times to eliminate germ cells.

The resultant Sertoli cell-enriched fraction was resuspended into 0.25 ml of media with the chromaffin cells for at least 24 hours before transplantation. During the day of transplantation, the solution containing the Sertoli cell-enriched fraction and the chromaffin cells were resuspended using a Pasteur pipet then suctioned by a Hamilton syringe with a spinal needle of gauge 20.

### Surgery

The surgical procedures were carried out in sterile conditions, as is well known in the art (Pakzaban et al., 1993). All animals were initially anesthetized with 0.60 ml/kg of sodium pentobarbital, then placed in Kopf stereotaxic instrument. Bilateral striatal transplants were performed with coordinates set at: anterioposterior = +1.2, medialateral = +/-2.8; dorsoventral - 6.0, 5.9 & 5.8 (based on the atlas of Paxinos and Watson, 1984). The right hemisphere of the brain was transplanted with bovine chromaffin cells while the left hemisphere received chromaffin cells plus Sertoli cells. Each side received a total volume of 3 µl of the cell cocktail solution (1 µl per DV site). After surgery, the animals were placed on heating pads until recovery. Animals received a short course of immunosuppression using Cyclosporine-A (20 mg/kg/d,i.p.) immediately after surgery and on the day following the transplant. All animals were sacrificed at one month post transplant.

### Histology

Animals were anesthetized with 0.70 ml/kg of sodium pentobarbitol, then perfused with 500 ml of 0.9% isotonic saline and 500 ml paraformaldehyde. The animals were then decapitated, and the brain removed and post-fixed overnight in 40% paraformaldehyde with 30% sucrose in PBS. The following day, brain sections were cut at 30 microns using the Vibroslice (Campden Instrument, UK). Host tissue immunologic response was analyzed using the Lectin method (see below). Three experimenters conducted independent qualitative comparisons of the left and right side of the brain in a blind-randomized manner.

### Lectin Method

Following vibrotome sectioning, brain sections were placed in 0.1% Triton X-100 for 15-30 minutes. The sections were then washed in 0.1M cationic PBS(pH 7.2), containing 0.1mM CaCl₂, 0.1mM MgCl₂. Incubation of sections was carried out in 20.0 µg/ml lectin made in cationic PBS at 4°C for two hours. Rinsing, three times in PBS was done prior to incubation with DAB. The DAB stock solution was made by dissolving 10mg DAB in 20 ml phosphate buffer (0.1 M, pH 7.2) and adding 0.5 ml of 1% CoCl₂ to DAB solution while stirring. Sections were first preincubated in DAB solution for 15 minutes. Upon adding 0.6 ml 3% H₂O₂ to 20.5 ml DAB solution, incubation lasted for five to ten minutes or until appropriate reaction was reached. Sections were again rinsed three times in PBS. Finally, sections were mounted from distilled water to wash out salts.

### RESULTS

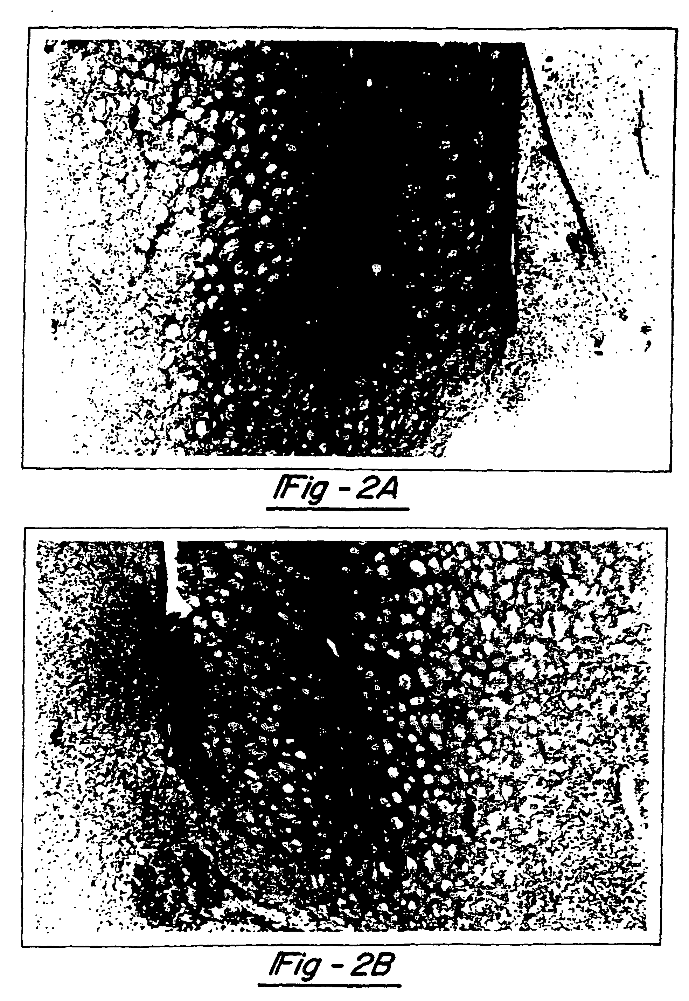

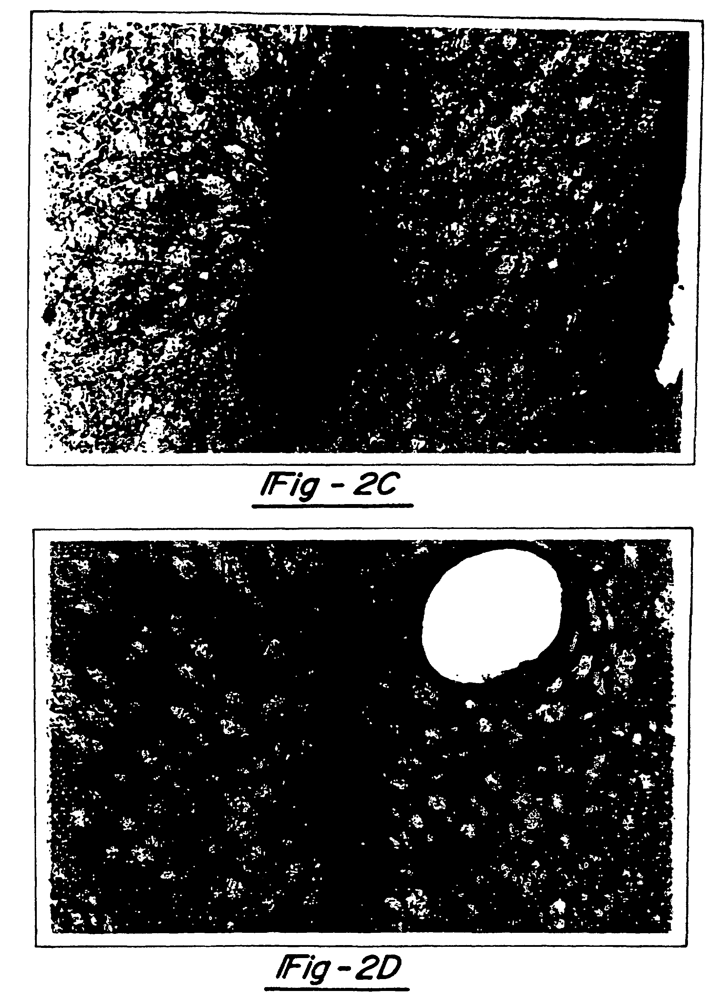

Histological analyses of both transplant sites revealed that the transplanted side with chromaffin cells alone had greater glial infiltration and higher number of macrophages than the transplanted side with chromaffin cells plus Sertoli cells (Figures 1 & 2). No significant difference in glial response was observed between animals treated with or without cyclosporine.

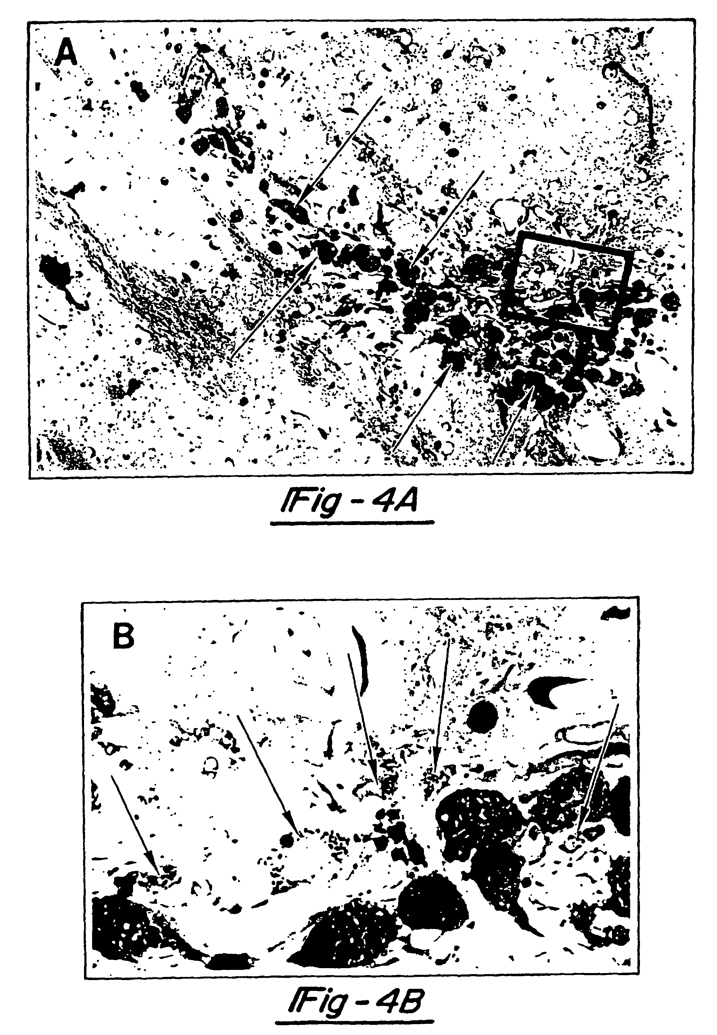

Two conclusions are made from the data: 1) a localized effect of the Sertoli cells which appeared to suppress the immune response of the host tissue to the transplanted cells, and 2) a sustained immunosuppression, with or without a short course of CsA administration, can be achieved with simultaneous transplantation of chromaffin cells with Sertoli cells (Figure 4).

It can therefore be concluded that Sertoli cells can provide an immunologically privileged site in the CNS by direct intracranial infusion. Furthermore, with significant immunosuppression obtained following xenografts in the present study, Sertoli cells can provide greater beneficial effects on creating immunologically privileged sites following allografts, and enhance survival and integration of the transplant through immunological and trophic support.

### EXAMPLE 2

Specific Protocol: The protocol involves three basic steps, Sertoli cell isolation, co-culture with neural specific cells or other appropriate cells and transplantation of the co-culture into the CNS (for details regarding the cell isolation see Selawry and Cameron (1993) and for details regarding cell transplantation see (Pakzaban et al., 1993).

### 1A. Sertoli cell isolation

The isolation procedure follows a well-defined method as described in reference (Selawry and Cameron, 1993). The cell culture medium used in all isolation steps and in which the cells were incubated was DMEM:Hams F12 supplemented with retinol, ITS and gentamicin sulfate (Cameron and Muffly, 1991). Testes were surgically collected from sixteen day old male Sprague-Dawley rats. The testes were decapsulated and prepared for enzymatic digestion to separate other testicular cell types from the Sertoli cells. The enzymatic procedure using collagenase (0.1%), hyaluronidase (0.1%) and trypsin (0.25%) is routinely used in many cell isolation protocols. After sequential enzymatic digestion, the Sertoli cell isolate was washed with culture medium, transferred to sterile cell culture vessels and placed in a humidified, 5% Co₂-95% air tissue culture incubator. Following forty-eight hours of pre-incubation in a 39°C incubator, the Sertoli cells were washed to remove any contaminating debris. The resultant Sertoli cell-enriched fraction was resuspended into 0.25 ml of DMEM/F12 medium, and incubated at 37°C for at least 24 hours.

The Sertoli cells were liberated from the vessel floor with 0.01% trypsin, transferred to a sterile conical test tube and repeatedly washed by centrifugation and then treated with trypsin inhibitor to cease the enzymatic action of trypsin. During the day of transplantation, the Sertoli cell-enriched fractions are resuspended and suctioned by a Hamilton syringe with a twenty gauge spinal needle.

### 1B. Isolation and Pretreatment of Sertoli Cells

As previously described (Cameron and Muffly, 1991) decapsulated rat testes were subjected to sequential enzymatic treatment at 37°C using 0.25% trypsin (Sigma) and 0.1% collagenase (Sigma, type V) (Cameron and Muffly, 1991). The resulting Sertoli cell aggregates were equally distributed in a volume of 20ml incubation medium into 75cm² tissue culture flasks (Costar). Plated Sertoli aggregates were incubated at 39°C in 5% CO₂-95% air for 48 hours after which cells were subjected to hypotonic treatment with sterile 0.5mM Tris-HCl buffer for one minute (Galdieri et al. 1981) to expedite the removal of contaminating germ cells. Following two washes with incubation medium, flasks were replenished with 20ml incubation medium and returned to the CO₂-injected incubator at 37°C in 5% CO₂-95% air. The resulting pre-treated Sertoli-enriched monocultures contained greater than 95% Sertoli cells. Plating density (< 2.0 X 10⁶ Sertoli cells/cm²) did not result in a confluent monolayer of cells.

### 2. Co-culture with neural specific cells

Sertoli cells and neural cell specific for transplantation for the neurodegenerative model were suspended by trypsin (0.01%), washed three times with medium and placed into a sterile cell culture vessel twenty-four hours prior to transplantation. The resulting co-culture was placed in a 5% CO₂-95% air incubator at 37°C until utilized for transplantation.

### 3. Transplantation of co-culture into the CNS

The transplantation protocol follows the procedure as previously described by Pakzaban et al., (1993). The animal surgery was carried out in sterile conditions. All animals were initially anesthetized with 0.60 ml/kg sodium pentobarbital, then placed in a Kopf stereotaxic instrument. For the Parkinson's disease model, unilateral striatal transplants are performed with coordinates set at: anterioposterior - +1.2, mediolateral - +/-2.9, dorsoventral - 6.0, 5.9 and 5.8 (based on the atlas of Paxinos and Watson) (1984). Different coordinates are used for different neurodegenerative animal models also based on Paxinos and Watson (1984). The striatum ipsilateral to the lesioned substantia nigra is transplanted with Sertoli cells or with the Sertoli cell co-culture. Each striatum receives a total volume of 3µl of Sertoli cell or co-culture suspension. One microliter of the cell suspension was infused over one minute per dorsoventral site. Another five minutes was then allowed upon reaching the last dorsoventral site before retracting the needle. After surgery, the animals were placed on heating pads until they recovered. The animals received a short course of immunosuppression therapy using Cyclosporine-A (20 mg/kg/d, i.p.) immediately after surgery and on the day following the transplant.

Sertoli cells and/or co-culture suspensions are transplanted into animal models of various neurodegenerative disorders by stereotaxic coordinates defined for the specific disorder, as illustrated in the Parkinson's disease example. All experimental animals are systematically assayed for functional recovery by techniques specific to that animal model.

### EXAMPLE 3

Figure 4A illustrates that electron dense chromaffin cells (arrows) were easily identified because of the inclusion of secretory granules unique to the cells. Figure 4B shows the boxed area in Figure 4A at a higher magnification, wherein at a higher resolution, Sertoli cells (arrows) were detected immediately adjacent to the electron dense chromaffin cells. This demonstrates the survival of co-grafted adrenal chromaffin cells with Sertoli cells in the brain.

### (1) Growth of neural cells

### Incubation Medium and Sertoli Cell Pre- Conditioned Medium:

The incubation medium used for Sertoli cell culture and co-culture was Dulbecco's Minimum Essential Medium:Hams F12 Nutrient Medium (Whittaker Bioproducts) mixed 1:1 and supplemented with 3mg/ml L-glutamine (Sigma, grade III), 0.01cc/ml insulin-transferrin-selenium (ITS, Collaborative Research, Inc.), 50 ng/ml retinol (Sigma), 19µl/ml lactic acid (Sigma) and 0.01cc/ml gentamicin sulfate (Gibco).

Following the first 48 hour incubation period of isolated Sertoli cells, media was collected and centrifuged at 1500rpm for five minutes. The supernatent was collected and immediately frozen in sterile test tubes. This medium was identified as Sertoli pre-conditioned medium (SCM).

### Isolation and Incubation of Fetal Brain Cells:

Fetal brain cells (FBC) were collected from the ventral mesencephalon of fetal rats (15-17 days gestation). The fetal brain tissue was suspended in medium and initially dispersed by passing it through a series of sequentially decreasing sized hypodermic needles (18-26 gauge). The resulting suspension was treated with 0.1% trypsin for five minutes and followed by 0.1% trypsin inhibitor for two minutes. The suspended FBC were washed (three times), resuspended in incubation medium and plated in poly-L-lysine-coated culture vessels.

Cells from the ventral mesencephalon of fetal rats (VM) were isolated and cultured for seven days in control medium (CM) or Sertoli cell pre-conditioned medium (SCM) as shown in Figure 3A, VM cells incubated in CM showed no evidence of cellular stimulation or differentiation. Referring to Figure 3B, VM cells incubated in SCM were highly stimulated. Figure 3C illustrates that at higher magnification, VM cells incubated in SCM show neurite outgrowth.

### EXAMPLE 4

**Incorporation of latex beads:** Sertoli cells were isolated and prepared for incubation as described. Prior to transplantation (approximately 12 hours), sterile 1µm latex beads (10µl/ml medium; Pelco, Tustin, CA) were added to the incubation medium. Sertoli cells rapidly phagocytosed the beads. Immediately prior to transplantation, the beaded Sertoli cells were washed (three times) and resuspended in 1ml of incubation medium.

Referring to Figure 5A, Sertoli cells were transplanted into the striatum of the brain wherein the penetration tract (arrows) and the site of Sertoli cell transplantation are shown. At higher magnification as shown in Figure 5B, Sertoli cells (arrows) were easily identified because of the inclusion of 1µ latex beads which were loaded into the Sertoli cells prior to transplantation.

### EXAMPLE 5

### Effects of Cyclosporine A (CsA) on the survival of transplanted Sertoli cells

**Fluorescent cell labeling:** Immediately prior to transplantation (approximately two hours), Sertoli cell monocultures were treated with CM-DiI fluorescent dye for cell tracking (100µl stock/ml medium; Molecular Probes, Inc., Eugene, OR) for 7 minutes at 37°C and then placed in the refrigerator (4°C) for an additional 15 minutes. Fluorescent "tagged" Sertoli cells were washed (three times) and resuspended in 1ml of incubation medium.

The effect of cyclosporine A on the survival of grafted Sertoli cells in situ was examined. Grafted Sertoli cells were labeled with a fluorescent tag (DiI) prior to transplantation into the striatum of the brain. The tissue was collected one month post-transplantation. Referring to Figure 6A, viable fluorescent Sertoli cells were seen in a rat host that had not received immunosuppression therapy with cyclosporine A. Referring to Figure 6B, viable fluorescent Sertoli cells are shown in a rat host that had not received cyclosporine A immunosuppression therapy. This example demonstrates that cyclosporine A is not necessary for the survival of Sertoli cells transplanted into the brain.

Throughout this application various publications are referenced by citation. Full citations for the publication are listed below.

The invention has been described in an illustrative manner, and it is to be understood the terminology used is intended to be in the nature of description rather than of limitation.

Obviously, many modifications and variations of the present invention are possible in light of the above teachings. Therefore, it is to be understood that within the scope of the appended claims, the invention may be practiced otherwise than as specifically described.

### REFERENCES CITED

Berden et al., "Severe central nervous system toxicity associated with cyclosporine" Lance 26:219-220 (1985).
Bjorklund and Stenevi, "Intracerebral neural grafting: a historical perspective" in Bjorklund, A. and U. Stenevi, eds. Neural grafting in the mamanalian CNS, Amsterdam: Elsevier, 3-11 (1985).
Bjorklund, "Dopaminergic transplants in experimental Parkinsonism: Cellular mechanisms of graft-induced functional recovery" Current Biology, 2:683-689 (1992).
Borlongan et al., "Cyclosporine-A increases spontaneous and dopamine agonist-induced locomotor behavior in normal rats" Cell Transplant., 4:65-73 (1995).
Borlongan et al. "PR: Systemic 3-nitropropionic acid: Behavioral deficits and striatal damage in rats", Brain Research Bulletin, 36:549-556 (1995).
Cameron et al., "Successful islet/abdominal testis transplantation does not require Leydig cells" Transplantation, 50:649-653 (1990).
Cameron and Muffly, "Hormonal regulation of spermated binding to Sertoli cells in vitro" J.Cell Sci., 100:532-533 (1991).
de Groen et al., "Central nervous system toxicity after liver transplantation" N. Engl. J. Med. 14:861-866 (1984).
Dunnett and Bjorklund, Functional Neural Transplantation, Advances in Neuroscience, Volume 2, Raven Press, New York.
Freeman et al., "The USF protocol for fetal nigral transplantation in Parkinson's disease" Experimental Neurology, 129:6-7 (1994).
Griswold, "Protein secretion by Sertoli cells: general considerations" in Russel, L.D. and M.D. Griswold, eds. The Sertoli Cell, Cache River Press, Clearwater, FL., 195-200.
Isacson et al., "Graft-induced behavioral recovery in an animal model of Huntington's disease" Proc. Natl. Acad. Sci., 83:2728-2732 (1986).
Koutouzis et al., "PR: Systemic 3-nitropropionic acid: Long term effects on locomotor behavior" Brain Research, 646:242-246 (1994).
Lindvall et al., "Transplantation in Parkinson's disease: two cases of adrenal medullary grafts to the putamen" Ann. Neurol., 22:457-468 (1987).
Lindvall et al., "Grafts of fetal dopamine neurons survive and improve motor function in Parkinson's disease" Science, 247:574-577 (1990).
Pakzaban et al., "Increased proportion of Ache-rich zones and improved morphological integration in host striatum of fetal grafts derived from the lateral but not the medial ganglionic eminence" Exp. Brain Res., 97:13-22 (1993).
Paxinos and Watson, "The rat brain in stereotaxic coordinates" Sydney, Academic Press (1984).
Sagen et al., "Transplants of immunologically isolated xenogeneic chromaffin cells provide a long-term source of pain-reducing neuroactive substances" J. Neurosci. 13:2415-2423 (1993).
Sanberg, PR. (Editor-in-chief) "Cell Transplantation", Elservie Science Publishers, New York, 1992-Present
Sanberg et al., "Cell transplantation for Huntington's disease" R.G. Landes Co., Boca Raton, FL, pp. 19-21 (1994).
Sanberg et al., "Sertoli cells induce immunoreactivity and functional recovery following transplantation into the striatum of 6-OHDA lesioned rats (in preparation) (1995).
Selawry and Cameron, "Sertoli cell-enriched fraction in successful islet cell transplantation" Cell Transplant., 2:123-129 (1993).
Wictorin et al., "Reformation of long axon pathways in adult rat CNS by human forebrain neuroblasts" Nature, 347:556-558 (1990).

## Claims

1. A composition containing Sertoli cells and neural or adrenal chromaffin cells.

2. A composition according to claim 1, wherein said composition contains Sertoli cells and neural cells.

3. A composition according to claim 1 or 2, wherein said neural cells are brain cells.

4. A composition according to any of claims 1 to 3, wherein said Sertoli cells have been co-cultured with said neural or adrenal chromaffin cells.

5. A composition according to any of claims 1 to 4, wherein said composition further contains trophic factors secreted *in situ* by said Sertoli cells.

6. A composition according to any of claims 1 to 5, wherein said Sertoli cells are porcine cells.

7. A composition according to any of claims 1 to 6, wherein said Sertoli cells are isolated.

8. Use of a composition containing Sertoli cells and neural or adrenal chromaffin cells for the manufacture of a medicament for use in producing a sustained localised immunosuppressive effect and trophic effect in the central nervous system of a subject, such as a human.

9. Use according to claim 8, wherein said composition contains Sertoli cells and neural cells.

10. Use according to claims 8 or 9, wherein said neural cells are brain cells.

11. Use according to any of claims 8 to 10, wherein said Sertoli cells have been co-cultured with said neural cells or adrenal chromaffin cells.

12. Use according to any of claims 8 to 11, wherein said composition further contains trophic factors secreted *in situ* by said Sertoli cells.

13. Use according to any of claims 8 to 12, wherein said Sertoli cells are porcine cells.

14. Use according to any of claims 8 to 13, wherein said Sertoli cells are isolated.

15. Use of a composition containing Sertoli cells and neural or adrenal chromaffin cells for the manufacture of a medicament for treating a neurological disorder.

16. Use according to claim 15, wherein said neurological disorder is selected from the group consisting of epilepsy, stroke, Huntington's disease, head injury, spinal injury, pain, Parkinson's disease, myelin deficiencies, muscular dystrophy, neurological pain, amyotrophic lateral sclerosis, Alzheimer's disease, and affective disorders of the brain.

17. Use according to claims 15 or 16, wherein said Sertoli cells have been co-cultured with said neural or adrenal chromaffin cells.

18. Use according to any of claims 15 to 17, wherein said composition contains Sertoli cells and neural cells.

19. Use according to any of claims 15 to 18, wherein said neural cells are brain cells.

## Patentansprüche

1. Mischung, beinhaltend Sertolizellen und neurale oder adrenale chromaffine Zellen.

2. Mischung nach Anspruch 1, wobei die Mischung Sertolizellen und neurale Zellen enthält.

3. Mischung nach Anspruch 1 oder 2, wobei die neuralen Zellen Gehirnzellen sind.

4. Mischung nach einem der Ansprüche 1, 2 oder 3, wobei die Sertolizellen mit den neuralen oder adrenalen chromaffinen Zellen gemeinsam kultiviert wurden.

5. Mischung nach einem der Ansprüche 1 bis 4, wobei die Mischung weiterhin von den Sertolizellen in situ sektretierte Nahrungsfaktoren enthält.

6. Mischung nach einem der Ansprüche 1 bis 5, wobei die Sertolizellen Schweinezellen sind.

7. Mischung nach einem der Ansprüche 1 bis 6, wobei die Sertolizellen isoliert sind.

8. Verwendung einer Mischung, beinhaltend Sertolizellen und neurale oder adrenale chromaffine Zellen, zur Herstellung eines Medikaments zur Anwendung in der Erzeugung einer dauerhaften lokalisierten immunsuppressiven Wirkung und einer trophischen Wirkung im zentralen Nervensystem eines Subjekts, wie etwa eines Menschen.

9. Verwendung nach Anspruch 8, wobei die Mischung Sertolizellen und neurale Zellen enthält.

10. Verwendung nach Anspruch 8 oder 9, wobei die neuralen Zellen Gehimzellen sind.

11. Verwendung nach einem der Ansprüche 8 bis 10, wobei die Sertolizellen mit den neuralen oder adrenalen chromaffinen Zellen gemeinsam kultiviert wurden.

12. Verwendung nach einem der Ansprüche 8 bis 11, wobei die Mischung weiterhin von den Sertolizellen in situ sektretierte Nahrungsfaktoren enthält.

13. Verwendung nach einem der Ansprüche 8 bis 12, wobei die Sertolizellen Schweinezellen sind.

14. Verwendung nach einem der Ansprüche 8 bis 13, wobei die Sertolizellen isoliert sind.

15. Verwendung einer Mischung, beinhaltend Sertolizellen und neurale oder adrenale chromaffine Zellen, zur Herstellung eines Medikaments zur Behandlung einer neurologischen Funktionsstörung.

16. Verwendung nach Anspruch 15, wobei die neurologische Funktionsstörung eine der nachfolgend aufgeführten ist: Epilepsie, Schlaganfall, Chorea Huntington, Kopfverletzungen, Rückenmarksverletzungen, Schmerzen, Parkinsonsche Krankheit, Myelinmangelzustände, Muskeldystrophie, neurologische Schmerzen, amyotrophe Lateralsklerose, Alzheimersche Krankheit und affektive Geistesstörungen des Gehirns.

17. Verwendung nach Anspruch 15 oder 16, wobei die Sertolizellen mit den neuralen oder adrenalen chromaffinen Zellen gemeinsam kultiviert wurden.

18. Verwendung nach Anspruch 15 bis 17, wobei die Mischung Sertolizellen und neurale Zellen enthält.

19. Verwendung nach Anspruch 15 bis 18, wobei die neuralen Zellen Gehirnzellen sind.

## Revendications

1. Composition contenant des cellules de Sertoli et des cellules chromaffines neurales ou surrénales.

2. Composition selon la revendication 1, dans laquelle ladite composition contient des cellules de Sertoli et des cellules neurales.

3. Composition selon la revendication 1 ou la revendication 2, dans laquelle lesdites cellules neurales sont des cellules cérébrales.

4. Composition selon l'une quelconque des revendications 1 à 3, dans laquelle lesdites cellules de Sertoli ont été co-cultivées avec lesdites cellules chromaffines neurales ou surrénales.

5. Composition selon l'une quelconque des revendications 1 à 4, dans laquelle ladite composition contient en outre des facteurs trophiques sécrétés in situ par lesdites cellules de Sertoli.

6. Composition selon l'une quelconque des revendications 1 à 5, dans laquelle lesdites cellules de Sertoli sont des cellules porcines.

7. Composition selon l'une quelconque des revendications 1 à 6, dans laquelle lesdites cellules de Sertoli sont isolées.

8. Utilisation d'une composition contenant des cellules de Sertoli et des cellules chromaffines neurales ou surrénales pour la fabrication d'un médicament destiné à être utilisé dans la production d'un effet immunosuppresseur localisé et prolongé et d'un effet trophique dans le système nerveux central d'un sujet, tel qu'un humain.

9. Utilisation selon la revendication 8, dans laquelle ladite composition contient des cellules de Sertoli et des cellules neurales.

10. Utilisation selon la revendication 8 ou la revendication 9, dans laquelle lesdites cellules neurales sont des cellules cérébrales.

11. Utilisation selon l'une quelconque des revendications 8 à 10, dans laquelle les cellules de Sertoli ont été co-cultivées avec lesdites cellules chromaffines neurales ou surrénales.

12. Utilisation selon l'une quelconque des revendications 8 à 11, dans laquelle ladite composition contient en outre des facteurs trophiques sécrétés *in situ* par lesdites cellules de Sertoli.

13. Utilisation selon l'une quelconque des revendications 8 à 12, dans laquelle lesdites cellules de Sertoli sont des cellules porcines.

14. Utilisation selon l'une quelconque des revendications 8 à 13, dans laquelle lesdites cellules de Sertoli sont isolées.

15. Utilisation d'une composition contenant des cellules de Sertoli et des cellules chromaffines neurales ou surrénales pour la fabrication d'un médicament destiné au traitement d'un trouble neurologique.

16. Utilisation selon la revendication 15, dans laquelle ledit trouble neurologique est choisi dans le groupe comprenant l'épilepsie, l'accident vasculaire cérébral, la maladie de Huntington, une lésion de la tête, une lésion rachidienne, la douleur, la maladie de Parkinson, les déficits myéliniques, la dystrophie musculaire, la douleur neurologique, la sclérose latérale amyotrophique, la maladie d'Alzheimer, et les troubles affectifs du cerveau.

17. Utilisation selon la revendication 15 ou la revendication 16, dans laquelle lesdites cellules de Sertoli ont été co-cultivées avec lesdites cellules chromaffines neurales ou surrénales.

18. Utilisation selon l'une quelconque des revendications 15 à 17, dans laquelle ladite composition contient des cellules de Sertoli et des cellules neurales.

19. Utilisation selon l'une quelconque des revendications 15 à 18, dans laquelle lesdites cellules neurales sont des cellules cérébrales.
